# EUROPÄISCHE PATENTSCHRIFT

(11) **EP 0 870 512 B1**
(45) Veröffentlichungstag und Bekanntmachung des Hinweises auf die Patenterteilung: **03.07.2002**
(21) Anmeldenummer: 98104796.2
(22) Anmeldetag: 17.03.1998
(51) Int. Cl.: A61L 2/26

(54) **Adapter für ein Sterilisationsgefäss in Form einer Kassette**
Adapter for a sterilisation case
Adaptateur pour cassette de stérilisation

(30) Priorität: 07.04.1997 DE 19714298
(43) Veröffentlichungstag der Anmeldung: 14.10.1998
(73) Patentinhaber: SciCan, Division of Lux & Zwingenberger, Toronto, Ontario M3B 3P9 (CA)
(72) Erfinder: Lemmen, Marcel, Mississauga, Ontario L4Z 3V1 (CA); Wu, Chris, Toronto, Ontario M6E 2W7 (CA)
(74) Vertreter: Borchert, Uwe Rudolf, Dipl.-Ing.

(56) Entgegenhaltungen:
- EP-A- 0 711 529
- DE-A- 3 232 329
- DE-A- 19 626 871

## Beschreibung

Die Erfindung betrifft ein Sterilisationsgefäß in Form einer in ein Sterilisationsgerät einschiebbaren Kassette gemäß der im Oberbegriff des Anspruches 1 angegebenen Art.

Wie die Erfahrung gezeigt hat, bereitet es besondere Schwierigkeiten insbesondere langgestreckte ärztliche und/oder zahnärztliche Instrumente mit Fluidkanälen wie Hand- und Winkelstücke nach deren Gebrauch zu reinigen und/oder zu sterilisieren, damit diese für den nächsten Gebrauch keimfrei einsetzbar sind. Das Reinigungsund/oder Sterilisationsfluid muss nämlich durch alle Hohlräume und Kanäle in ausreichender Weise geführt werden.

Eine weitere Schwierigkeit ergibt sich aus der Tatsache, dass anstelle großvolumige Sterilisationskammern aufweisenden und damit viel Energie verzehrenden Autoklaven nunmehr Sterilisationsgeräte in Gebrauch sind, deren Sterilisationsräume als in das Sterilisationsgerät einschiebbare zweiteilige ein Unter- und ein Oberteil umfassende Kassetten ausgebildet sind, deren geringes Volumen ein kostengünstiges wenig Zeit beanspruchendes Arbeiten ermöglicht.

Aus der DE 32 32 329 ist eine Spülmaschine zur Reinigung und Desinfektion von zahnärztlichen Instrumenten bestehend aus einem Gehäuse und lösbaren, einschiebbaren Fluidzu- und abführöffnungen aufweisenden, korbartigen Aufnahmeelementen bekannt. Ein Fluidverteiler in Form einer Palette ist an einen der Steckzapfen des Aufnahmeelements durch ein Anschlusselement anschließbar. Diese lösbare Verbindung stellt durch einen Verteilhohlraum eine Fluidzuführung dar. Der Fluidverteiler ist lösbar einsetzbar und die verschiedenen Steckzapfen sind zur Instrumentenaufnahme vorgesehen.

Auch aus der EP 0 638 297 A1 ist ein Sterilisationsgerät zur Aufnahme solcher Sterilisationsgefäße in Form von Kassetten bekannt geworden, die für die Aufnahme von langgestreckten ärztlichen und/oder zahnärztlichen Instrumenten mit Fluidkanälen mehrere Adapter aufweisen. Jeder dieser Adapter enthält einen Anschlußzapfen, der baulich auf die Konstruktion des Instrumentes abgestimmt ist. An dem dem Instrument abgewandten Ende ist jeder Adapter so ausgebildet, dass er an der Rückwand des Kassettenunterteils leicht lösbar befestigt werden kann, wozu eine Rastnase vorgesehen ist, die mit einer Ringnut eines Flansches an der Rückwand des Kassettenunterteils zusammenwirkt. In jedem Adapter befindet sich ein verschiebbarer Schaltplunger, der beim Aufschieben eines Instrumentes auf den Adapter entgegen der Kraft einer Feder betätigbar ist und dabei die benötigten Fluidkanäle frei gibt.

Eine solche Ausbildung ist aufwendig und erfordert Vorkehrungen an der Kassette selbst, so dass Kassetten unterschiedlicher Bauart erforderlich werden, sowie bereits vorhandene Kassetten mit solchen Adaptern nicht nachträglich ausgerüstet werden können.

Hier setzt nun die Erfindung ein, deren Aufgabe es ist, eine wohlfeile wenig Bauteile umfassende Adapter-Vorrichtung für Kassetten unterschiedlicher Bauart zu schaffen, mit der solche als Sterilisationsgefäße dienende Kassetten für die Aufnahme von Hohlräumen und Fluidkanäle aufweisenden Instrumenten auf einfache Weise betriebsicher arbeitend ausrüstbar sind, ohne dass hierfür die Kassetten selbst zu ändern oder besonders auszubilden sind.

Diese Aufgabe ist gemäß der Erfindung dadurch gelöst, dass ein Adapter vorgesehen ist, der als ein mehrere Instrumentenaufnahmen aufweisender in die Kassette quer zu deren Längserstreckung lösbar einsetzbarer Fluidverteiler ausgebildet ist, der über eine Schlauchverbindung mit einem innerhalb der Kassette in deren Fluidzuführung lösbar einsetzbaren Fluidabgriff fluidisch verbunden ist.

Nach einem weiteren Merkmal der Erfindung weist sowohl der Verteiler als auch der Fluidabgriff Klemmglieder zur lösbaren Verbindung mit der Kassette und deren Fluidzuführung auf.

Hierzu ist der Fluidverteiler erfindungsgemäß als ein eine stirnseitige Öffnung aufweisender im Querschnitt etwa rechtwinkliger Quader ausgebildet, an dessen Grundfläche Klemmstücke und an dessen im rechten Winkel dazu befindlichen Seitenfläche die Instrumentenaufnahmen angeformt sind, welche jeweils eine trichterförmige Aufnahmeöffnung und eine zylindermantelförmige Außenwandung aufweisen, wobei der Fluidabgriff einen auf einer eine Federklamme aufweisenden Stützplatte angeordneten Leitungsstutzen und ein der fluidführenden Dichtung der Kassette zugeordnetes Einsatzstück umfasst, das mit dem Leitungsstutzen kommuniziert.

Durch die erfindungsgemäße Ausbildung der Adaptervorrichtung wird das Reinigen und Sterilisieren von Öffnungen aufweisenden und mit Fluidkanälen versehenen Instrumenten, wie zahnärztlichen Handstücken, chirurgischen Endoskopieinstrumenten, medizinischem Schlauchmaterial und ähnlichem wesentlich erleichtert.

Durch die Art der Ausbildung der Adaptervorrichtung, insbesondere durch die Verwendung eines mit der Kassette lösbar mittels einer Klemmverbindung zu verbindenden, quer zur Längserstreckung einsetzbaren Adapters, der mehrere Aufnahmeöffnungen für das steckbare Einfügen von zu reinigenden und zu sterilisierenden Instrumenten aufweist, können sowohl vorhandene Kassetten als auch neu zu erwerbende Kassetten auf einfache Weise umgerüstet werden für das Sterilisieren langgestreckter Instrumente oder können zum Sterilisieren der üblichen eine geringe Längenausdehnung aufweisenden ärztlichen Instrumente wie Spritzen und ähnlichem nach dem Herausnehmen der Adapter-Vorrichtung gleichgut verwendet werden.

Die Erfindung ist nachfolgend anhand eines in der Zeichnung dargestellten Ausführungsbeispiels beschrieben.

Im einzelnen zeigen:
- Fig. 1: in perspektivischer Darstellung eine aus Unter- und Oberteil bestehende Kassette als Sterilisationsgefäß für ärztliche und/oder zahnärztliche Instrumente mit einer eingesetzten mehrteiligen Adapter-Vorrichtung gemäß der Erfindung,
- Fig. 2: in perspektivischer Darstellung den Fluidverteiler der Adapter-Vorrichtung nach Fig. 1,
- Fig. 3: einen Schnitt durch den Fluidverteiler nach Fig. 2,
- Fig. 4: in perspektivischer Darstellung der Fluidabgriff der Adapter-Vorrichtung nach Fig. 1 und
- Fig. 5: einen Schnitt durch den Fluidabgriff gemäß Fig. 4.

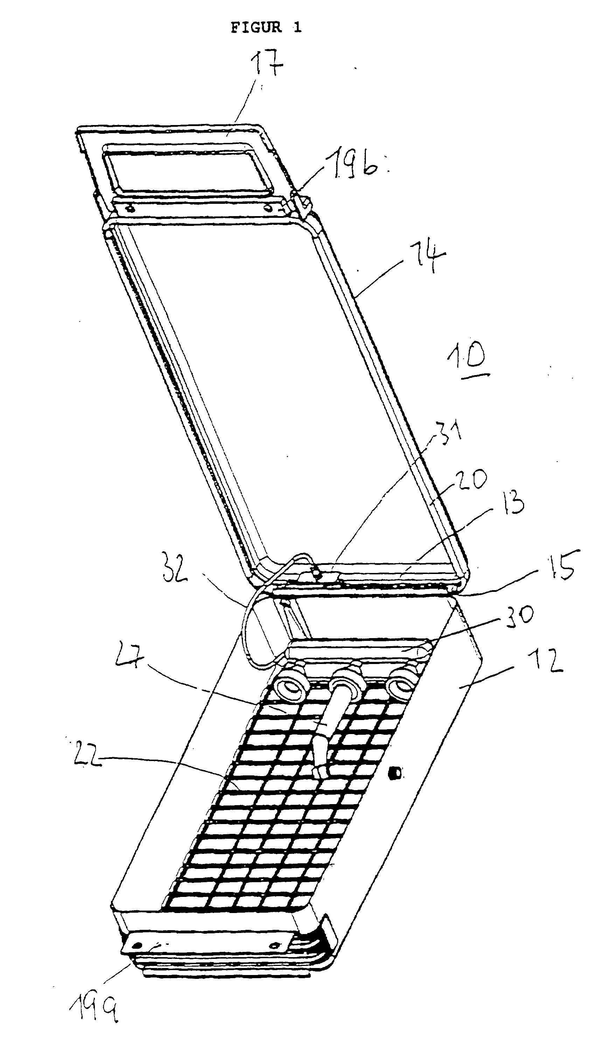

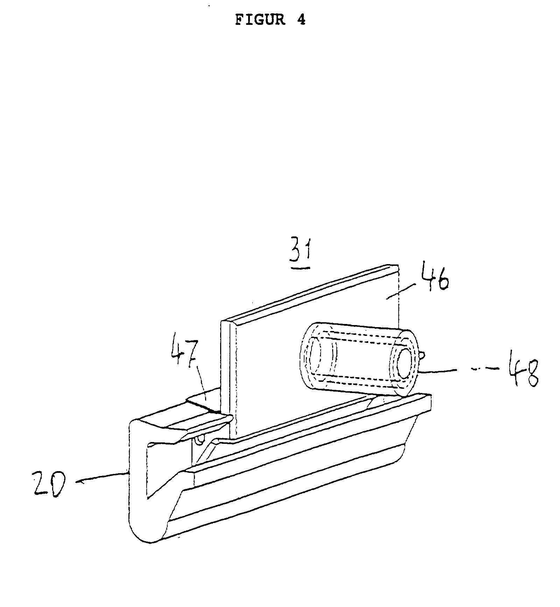

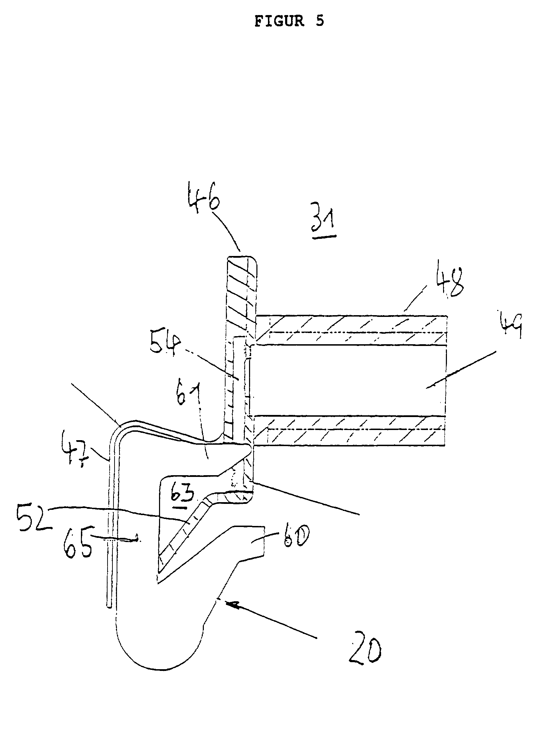

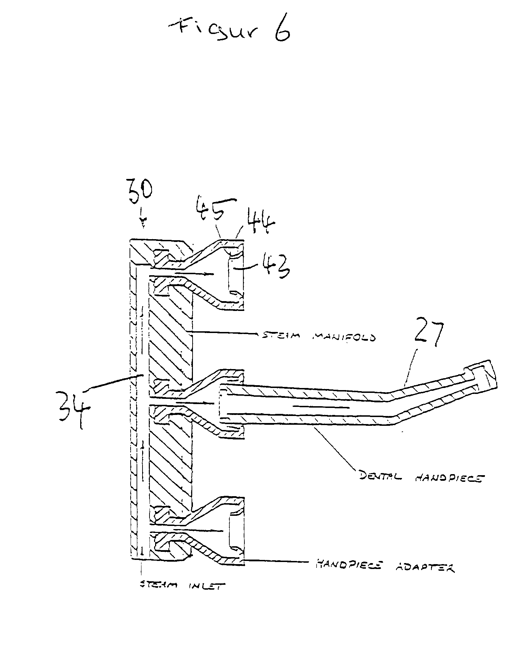

Ein Sterilisationsgefäß in Form einer in ein hier nicht dargestelltes Sterilisationsgerät - wie es z.B. in der EP 0 429 960 A2 beschrieben ist - einschiebbare Kassette 10 umfasst - wie Fig. 1 zeigt - ein Unterteil 12 und ein Oberteil 14, die über eine Scharnierverbindung 15 aufschwenkbar miteinander verbunden sind. Über einen Handgriff 17 am Oberteil 14 können Unter- und Oberteil vor dem Einschieben in das nicht dargestellte Sterilisationsgerät druckdicht miteinander verschlossen werden. Hierzu dient ein teilweise am Unterteil und teilweise am Oberteil angebrachter Verschluss 19a/19b sowie eine längs des Randes der Oberteils 14 eingelassene Dichtung 20, deren ungleichschenkliger etwa U-förmiger Querschnitt aus den Fig. 4 und 5 deutlich zu erkennen ist.

Auf den Boden des Unterteils der Kassette 10 liegt ein Gitterrost 22 als Auflage für die zu reinigenden und/oder zu sterilisierenden ärztlichen und/oder zahnärztlichen Instrumente. Die Kassette 10 weist ferner Zu- und Abführöffnungen 24 und 25 für den Eintritt und den Austritt des Reinigungs- und/oder Sterilisationsfluides z.B. Dampf auf.

Die beschriebene Kassette ist bekannt und im Handel erhältlich.

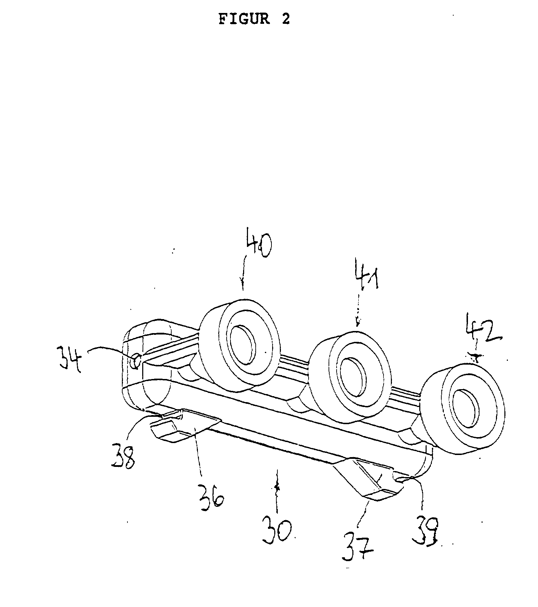

Zur funktionsgerechten Aufnahme von Instrumenten mit Hohlräumen und Fluidkanälen wie z.B. zahnärztliche Winkelstücke 27 ist eine Adapter-Vorrichtung vorgesehen, die aus einem Fluidverteiler 30, einem Fluidabgriff 31 und einen beide Teile fluidisch verbindenden Schlauch 32 besteht; Fluidverteiler und Fluidabgriff sind im Einzelnen in den Fig. 2 bis 5 dargestellt.

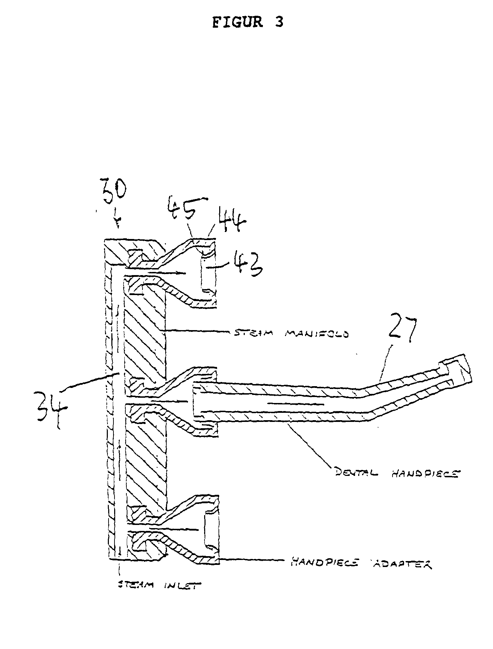

Der in den Fig. 2 und 3 dargestellte Fluidverteiler 30 ist ein aus hitzebeständigem Kunststoff bestehender quaderförmiger Körper mit einem zentralen Fluidkanal 34. An den Fluidverteiler an dessen dem Gitterrost 22 zugewandter Seite, also seiner Grundfläche sind im Abstand voneinander und gegeneinander ausgerichtet zwei integrale klemmglieder 36 und 37 angeformt, die im eingesetzten Zustand in der Kassette - siehe Fig. 1 - mit ihren Widerlagerflächen 38 und 39 zugeordnete Gitterstäbe des Gitterrostes 22 umfassen.

An der im rechten Winkel zur Grundfläche des Fluidverteilers angeordneten Seitenfläche sind drei Instrumente-Aufnahmen 40, 41 und 42 angeformt, deren zentrale Öffnungen mit dem Fluidkanal 34 kommunizieren, siehe Fig. 3.

Wie insbesondere die Fig. 2 und 3 zeigen, sind die Instrumente-Aufnahmen tüllenförmig ausgebildet und weisen jeweils eine trichterförmige Aufnahmeöffnung 43 und eine zylindermantelförmige Außenwandung 44 auf, die jeweils elastisch ausgebildet und nach innen zu einer klemmenden Mantelfläche 45 umgeformt ist. Auf diese Weise wird beim Einsetzen eines Instrumentes, wie z.B. des Winkelstückes 27, eine klemmende fluiddichte Verbindung zwischen der Instrumenten-Aufnahme und dem Instrument erzielt; vgl. Fig. 3 Mitte. Infolge der elastischen Ausbildung der Instrumente-Aufnahmen können Instrumente unterschiedlichen Durchmessers gleich gut aufgesteckt werden.

Der Fluidabgriff an der einen Fläche 31 besteht aus einer Stützplatte 46 aus rostfreiem Stahl, an die an der einen Fläche eine gebogene Federklammer 47 angeformt ist und die an der gegenüberliegenden Fläche einen Leitungsstutzen 48 trägt. An der einen Stirnseite der Stützplatte 46 ist ein winkelförmiger Einsatz-Steg 52 angeformt, der im Zusammenwirken mit der noch zu beschreibenden Dichtung 20 einen Fluidkanal 63 begrenzt, der über den Fluidkanal 54 in der Stützplatte 46mit der Öffnung 49 des Leitungsstutzen 48 kommuniziert; siehe Fig. 5.

Über den von den Schenkeln 60 und 61 der Dichtung 20 und vom unteren Randbereich 13 des Oberteils 14 der Kassette 10 eingeschlossenen Raum 63 erfolgt bei Kassetten der hier in Frage stehenden Art die Zuführung des oder der Fluide. Durch Einsetzen des beschriebenen Fluidabgriffes 31 auf das Oberteil 14 der Kassette 10, z.B. im Bereiche des Scharniers 15 - wie in den Fig. 1 und 5 dargestellt - werden der Schenkel 61 und die Basis 63 der Dichtung 20 federnd umfasst, so dass eine Fluidverbindung zum Kanal 54 und damit zum Leitungsstutzen 48 hergestellt wird. Über den Schlauch 32, der ebenfalls aus einem hitzebeständigen Kunststoff besteht und an seinen Enden mit der Öffnung des Fluidkanals 34 des Fluidverteilers und mit dem Leitungsstutzen 48 zusammenwirkende klemmende Endstücke trägt, erfolgt die Weiterleitung des zugeführten Fluids über den Fluidverteiler 30 zu den in die Instrumente-Aufnahmen 40 bis 42 eingesteckten Instrumente, hier zu dem Winkelstück 27, wie dies die Fig. 3 zeigt.

Da die einander zugewandten Flächen sowohl von Feder und Einsatzsteg als auch von fluidführender Dichtung miteinander korrespondierend ausgebildet sind, entsteht auf diese Weise eine fluiddichte Verbindung zwischen den beschriebenen miteinander kommunizierenden Bauteilen. Infolge der gewählten Art der Fluidzuführung wird die Funktionsweise der beschriebenen Vorrichtung auch dann aufrechterhalten, wenn lediglich ein Instrument wie in Fig. 1 dargestellt, in den Fluidverteiler eingesetzt ist.

Die Funktion der Kassette als Sterilisationsgefäß wird also durch die beschriebene Adapter-Vorrichtung in keiner Weise behindert oder irgendwie ungünstig beeinflusst. Die beschriebenen Klemmverbindungen von Fluidverteiler und Fluidabgriff ermöglichen ein überaus einfaches Einsetzen und Entfernen der Adapter-Vorrichtung.

## Patentansprüche

1. Sterilisationsgefäß in Form einer in ein Sterilisationsgerät einschiebbaren, Fluidzu- (63) und -abführöffnungen aufweisenden Kassette (10) mit einem Unter- und einem Oberteil zur funktionsgerechten Aufnahme von zu reinigenden und/oder sterilisierenden ärztlichen und/oder zahnärztlichen Instrumenten (27) mit Fluidkanälen, **dadurch gekennzeichnet, dass** ein Adapter vorgesehen ist, der als ein mehrere Instrumenten-Aufnahmen (40, 41, 42) aufweisender, in die Kassette (10) quer zu deren Längserstreckung lösbar einsetzbarer Fluidverteiler (30) ausgebildet ist, der über eine Schlauchverbindung (32) mit einem innerhalb der Kassette (10) in deren Fluidzuführung (63) lösbar einsetzbaren Fluidabgriff (31) verbunden ist.

2. Sterilisationsgefäß nach Anspruch 1, **dadurch gekennzeichnet, daß** der Fluidverteiler (30) klemmglieder (36, 37) und der Fluidabgriff (31) eine Federklammer (47) zur lösbaren Verbindung mit der Kassette (10) und deren Fluidzuführung (63) aufweisen.

3. Sterilisationsgefäß nach den Ansprüchen 1 und 2, **dadurch gekennzeichnet, dass** der Fluidverteiler (30) ein einen stirnseitig offenen Fluidkanal (34) aufweisender, im Querschnitt etwa rechtwinkliger Quader ist, an dessen Grundfläche Klemmglieder (36, 37) und an dessen im rechten Winkel dazu befindlichen Seitenfläche die Instrumentenaufnahmen (40, 41, 42) angeformt sind, welche jeweils eine trichterförmige Aufnahmeöffnung (43) und eine zylindermantelförmige Außenwandung (46) aufweisen.

4. Kassette nach den Ansprüchen 1 und 2, **dadurch gekennzeichnet, dass** der Fluidabgriff (31) einen auf einer eine Federklammer (47) aufweisenden Stützplatte (46) angeordneten Leitungsstutzen (48) und ein der fluidführenden Dichtung (20) der Kassette (10) zugeordnete Einsatzsteg (52) umfasst zwecks Bildung einer mit dem Leitungsstutzen (48) kommunizierenden Fluidverbindung.

5. Kassette nach Anspruch 4, **dadurch gekennzeichnet, dass** die Stützplatte (46) eine ebene Platte ist, und dass die einander zugewandten Flächen sowohl von Feder und Einsatzstück als auch von fluidführender Dichtung miteinander korrespondierend ausgebildet sind.

6. Kassette nach den Ansprüchen 2 bis 5, **dadurch gekennzeichnet**, das die Klemmglieder (36, 37) des Fluidverteilers (30) als integrale mit zugeordneten Stäben einer Gitterrosteinlage (22) der Kassette (10) korrespondierende Widerlager des Fluidverteilers (30) ausgebildet sind.

## Claims

1. Sterilization vessel of the cassette type (10) capable to be inserted into a sterilization apparatus and comprising apertures (63) for dispensing and withdrawing fluids and an upper and lower part to receive according to their functional use medical or dental instruments with fluid conduits which have to be cleaned and/or sterilized, **characterized in that** an adapter is provided designed as a manifold (30) which is removably located inside the cassette (10) transverse to its longitudinal direction and is provided with a plurality of sockets (40, 41, 42) that are adapted to engage with the instruments, said adapter being connected via a flexible tubing (32) to a fluid tap (31) which is removably attached inside the cassette to the fluid supply aperture (63) of the manifold.

2. Sterilization vessel according to claim 1, **characterized in that** the fluid manifold (30) is provided with clips (36, 37) and the fluid tap (31) with a spring clamp (47) for demountably connecting the manifold to the cassette (10) and its fluid supply aperture (63).

3. Sterilization vessel according to claims 1 and 2, **characterized in that** the manifold (30) is similar in form to a rectangular parallelepiped which is provided with a fluid channel (34) open at its lateral end and has moulded-in clips (36, 37) at its bottom and integrally mounted sockets (40, 41, 42) at its vertical side faces, the sockets each presenting a funnel shaped opening (43) and a tubular outer wall (46).

4. Cassette according to claims 1 and 2, **characterized in that** the fluid tap (31) includes a threaded stud (48) mounted to a backing plate (46) having a spring clamp (47) as well as a partition wall (52) associated to the fluid seal (20) of the cassette (10) so as to establish fluid connection with the stud (48).

5. Cassette according to claim 4, **characterized in that** the base plate (46) is a flat plate and that the opposite faces of the spring clamp and the partition as well as of the fluid seal are correspondingly shaped.

6. Cassette according to claims 2 to 5, **characterized in that** the moulded-in clips (36,37) of the manifold (30) form abutments for the respectively associated rods of a gridlike insert (22) of the cassette (10).

## Revendications

1. Récipient sous la forme d'une cassette (10) destinée à être insérée dans un autoclave de stérilisation et comportant des orifices d'admission et de décharge (63) pour des fluides et une partie inférieure et haute destinées à recevoir selon la fonction recherchée des instruments (27) canalaires médicales et/ou dentaires à nettoyer et/ou à stériliser, **caractérisé en ce que** un adaptateur est prévu qui peut être placé à l'intérieur de la cassette (10) de manière détachable et en travers de la direction longitudinale, et que cet adaptateur se présente sous forme d'un distributeur de fluides (30) avec plusieurs dispositifs de raccordement (40, 41, 42) pour les instruments et qui par l'intermédiaire d'un tuyau flexible (32) est relié à une prise de fluide (31) disposée de manière détachable à l'intérieur de la cassette et en communication avec l'ensemble d'admission de fluide (63).

2. Récipient de stérilisation selon la revendication 1, **caractérisé par le fait que** le distributeur de fluide (30) comporte des éléments de serrage (36, 37) et la prise de fluide (31) est munie d'une pince à ressort (47) pour le raccordement détachable avec la cassette (10) et son ensemble d'admission de fluide (63).

3. Récipient de stérilisation selon les revendications 1 et 2, **caractérisé en ce que** le distributeur de fluide (30) se présente,(en viron), comme un parallelépipède droit avec un canal de fluide (34) débouchant vers sa face latérale et que les élements de serrage (36, 37) sont monolitiques avec sa base et que les dispositifs de raccordement pour les instruments (40, 41, 42) sont formés intégralement sur les faces latérales perpendiculaires, ces dispositifs de raccordement comportant chacun un logement (43) en forme d'entonnoire, dont l'enveloppe extérieure (46) est cylindrique.

4. Cassette selon les revendications 1 et 2, **caractérisé en ce que** la prise de fluide (31) comporte un raccord tubulaire (48) disposé sur une plaque de support (46) munie d'une pince à ressort (47) et un cloison de refend insérable (52) associé au conduit d'étanchéité (20) de la cassette (10) afin d'établir la communication de fluide avec le raccord tubulaire (48).

5. Cassette selon la revendication 4, **caractérisé en ce que** la plaque de support (46) est plane et que les faces en vis à vis respectivement de la pince à ressort et du cloison de refend ainsi que du conduite d'étanchéité sont concues de manière correspondante.

6. Cassette selon les revendications 2 à 5, **caractérisé en ce que** les éléments de serrage (36, 37) sont intégrés dans le distributeur de fluide (30) et contre lesquels s'appuient les barreaux correspondants d'une grille (22) insérée dans la cassette (10).
